(19) Europäisches Patentamt
European Patent Office
Office européen des brevets

(11) Publication number: 0 067 689 B1

# (12) EUROPEAN PATENT SPECIFICATION

(45) Date of publication of patent specification: **10.09.86**

(51) Int. Cl.[4]: **G 03 C 7/34** // C07C127/19

(21) Application number: **82303047.3**

(22) Date of filing: **11.06.82**

## (54) Cyan couplers and colour photographic materials containing them.

(30) Priority: **11.06.81 JP 90334/81**
**11.06.81 JP 90335/81**
**11.06.81 JP 90336/81**

(43) Date of publication of application:
**22.12.82 Bulletin 82/51**

(45) Publication of the grant of the patent:
**10.09.86 Bulletin 86/37**

(84) Designated Contracting States:
**DE FR GB**

(56) References cited:
**EP-A-0 028 099**
**FR-A-2 125 969**
**US-A-3 446 622**

(73) Proprietor: **KONISHIROKU PHOTO INDUSTRY CO. LTD.**
**No. 26-2, Nishishinjuku 1-chome Shinjuku-ku**
**Tokyo 160 (JP)**

(72) Inventor: **Sato, Ryosuke**
**5995 Hino, Hino-shi**
**Tokyo (JP)**
Inventor: **Kato, Katsunori**
**2-12-21 Nakanosanomachi**
**Hachioji-shi Tokyo (JP)**
Inventor: **Sasaki, Takashi**
**10-401 Takahatadai-Danchi 850 Misawa**
**Hino-shi Tokyo (JP)**
Inventor: **Sugita, Hiroshi**
**2-103 Famiiru-Nishihachioji 5-26 Sandamachi**
**Hachioji-shi Tokyo (JP)**

(74) Representative: **Ellis-Jones, Patrick George Armine et al**
**J.A. KEMP & CO. 14 South Square Gray's Inn**
**London WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 067 689 B1

## Description

This invention relates to a silver halide photosensitive material for color photography containing a novel cyan dye image-forming coupler.

A color image is usually formed by oxidative coupling of the oxiidation product, which is formed by reduction of the exposed silver halide grains with an aromatic primary amine type color developing agent, with a coupler capable of forming yellow magenta or cyan dye in a silver halide emulsion.

As the coupler which is typically employed for forming the cyan dye, there may be mentioned phenols and naphthols. The basic properties required for the phenols used as couplers are that the dye obtained has good spectral absorption characteristics, i.e., the spectrum has no absorption in green region, but sharp absorption, that the dye formed shows sufficient fastness to light, heat, moisture and the like as well as good colorability, i.e., the dye shows sufficient color sensitivity and density and further that there is no loss of the dye even if the bleaching or bleach-fix bath used containing EDTA iron (III) complex as main ingredient becomes exhausted.

Moreover, it has been a serious problem in order to avoid environmental pollution to remove the benzyl alcohol incorporated into a color developing agent. However, the present situation is that a satisfactory color development can not be generally accomplished unless benzyl alcohol is added. A particularly remarkable reduction in color development can be seen with a phenol cyan coupler when benzyl alcohol is removed. From this point of view, there is a demand for a phenol cyan coupler with higher color development even if benzyl alcohol is not present.

In order to meet the aforesaid requirements, various studies have been made, but there has not yet been found a coupler capable of perfectly meeting all the foregoing properties. For instance, as disclosed in U.S. Patent No. 2,801,171, 6-[α-(2,4-di-tert amylphenoxy)butaneamido]-2,4-di-chloro-3-methylphenol shows a good fastness to light, but has poor heat fastness, a great loss of the dye in an exhuasted bleach-fix bath as well as a large color reliance on benzyl alcohol and difficult removal of benzyl alcohol from the color developing agent. U.S. Patent No. 2,895,826 disclosed 2-heptafluorobutaneamido-5-[α(2,4-di-tert-amylphenoxy)hexaneamido]phenol, which shows excellent heat fastness and lack of loss of dye in an exhausted bleach-fix bath, but has inferior light fastness and color development. The coupler disclosed in Japanese Patent Laid-open Application No. 53—10963 encounters problems about removal of benzyl alcohol and also light fastness. Further, those phenol type cyan couplers disclosed in U.S. Patent No. 3,839,044, Japanese Patent Laid-open Application No. 47—37425, Japanese Patent Publication No. 48—36894, Japanese Patent Laid-open Applications No. 50—10135, No. 50—117422, No. 50—130441, No. 50—108841 and No. 50—120334 are regarded as unsatisfactory with regard to heat fastness and removal of benzyl alcohol. The phenol couplers having a ureido group at the 2-position thereof disclosed in British Patent No. 1,011,940 and U.S. Patents No. 3,446,622, No. 3,996,253, No. 3,758,308, No. 3,880,661 and the like tend to form cyan dyes which show broad spectral absorption and, further, considerable absorption within the green region of the spectrum due to the maximum absorption in the red region or a relatively short wave range, which seems to be unfavourable in color reproduction. Other phenol couplers having a ureido group at the 2-position thereof as disclosed in Japanese Patent Laid-openn Application No. 56—65134 (equivalent to European A—0028099) shows a considerably improved green absorption, but are still unsatisfactory in other respects.

We have made strenuous efforts to improve this situation and, as a result, have found that the couplers defined below substantially meet the above-recited characteristics required for a phenol cyan coupler.

More specifically, the silver halide photosensitive material for color photography according to the present invention is characterized by comprising a phenol type cyan coupler having the formula [I]

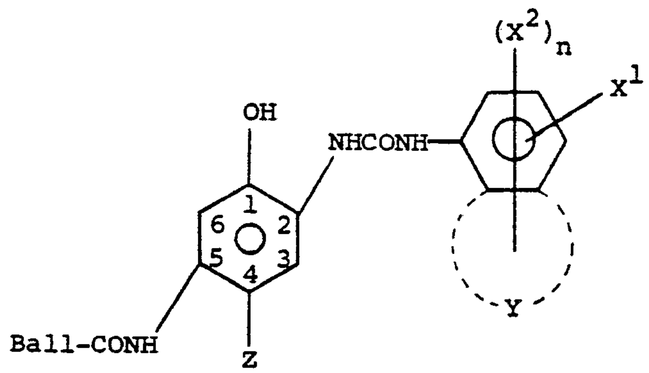

[I]

wherein

$X^1$ is $—COOR^1$, $—COR^1$, $—SO_2OR^1$, $—SO_2R^1$, 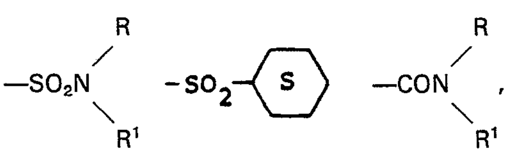 ,

—$NO_2$ or —$CF_3$, in which $R^1$ is an optionally substituted alkyl or aryl groups and R is a hydrogen atom, or an optionally substituted alkyl, or aryl group; or R and $R^1$ together form a 5 or 6 membered ring;
$X^2$ is a halogen atom, a hydroxy group, a nitro group or an optionally substituted alkyl, alkylcarbonyloxy, arylcarbonyloxy, alkoxy, aryloxy, alkylcarbonyl or arylcarbonyl group;
Y, when present, is a group of non-metallic atoms which completes a 5- or 6-membered condensed ring;
Z is a hydrogen atom or an atom or group removable upon coupling with an oxidation product of a color developing agent;
Ball is a ballast group; and
n is 0 or an integer from 1 to 4 inclusive such that, when n is 2 or more, each $X^2$ may be the same or different.

As the alkyl group in the definition of R and $R^1$ of the group $X^1$, there may be mentioned a $C_1$ to $C_{10}$ straight or branched alkyl group, preferably $C_1$ to $C_4$ straight or branched alkyl group. As the aryl group, there may be mentioned for example, a substituted or unsubstituted phenyl or naphthyl group.

In the above formula, $X^2$ represents an optionally substituted alkyl group, an alkylcarbonyloxy or arylcarbonyloxy group, an alkoxy group, an alkylcarbonyl or arylcarbonyl group or an alkoxy group. As the alkyl group, there is preferably mentioned a $C_1$ to $C_5$ straight or branched alkyl group; illustrative examples thereof include methyl, ethyl, isopropyl, butyl, tert-phenyl, chloromethyl, acetonyl and phenethyl.

As the alkylcarbonyloxy or arylcarbonyloxy group, there may be mentioned a $C_1$ to $C_5$ alkylcarbonyloxy group and an arylcarbonyloxy group such as acetoxy, propionyloxy, pivaloyloxy, benzoyloxy and naphthoyloxy.

As the alkoxy or aryloxy group, there is preferably mentioned a $C_1$ to $C_5$ alkoxy group and an aromatic alkoxy group; illustratiive examples thereof include methoxy, tert-butoxy, ethoxy-methoxy, substituted or unsubstituted phenoxy.

As the alkylcarbonyl or arylcarbonyl group, there is preferably mentioned a $C_1$ to $C_5$ alkylcarbonyl group and an arylcarbonyl group such as acetyl, pivaloyl, acetoacetyl, benzoyl, naphthoyl and toluyl.

Also, the phenyl group of the phenylureido portion in the coupler may have a condensed ring formed by the group Y. Where the said phenyl group has the condensed ring, the substituent $X^2$ may be located on the phenyl moiety or the said condensed ring moiety. The condensed ring formed by the group Y may be, for example, naphthalene, quinoline, benzothiophene, benzofuran or isocoumaran.

As illustrative examples of the group Z which may be removed in a coupling reaction, there may be mentioned, for example, a halogen atom, e.g., chlorine, bromine or fluorine atom; an aryloxy group, a carbamoyloxy group, a carbamoylmethoxy group, an alkylcarbonyloxy or arylcarbonyloxy group, a sulfonamido group, a succinimido group, oxygen or nitrogen atom being attached directly to the coupling site in the said groups. Further examples thereof include those disclosed in U.S. Patent No. 3,471,563, Japanese Patent Laid-open Application No. 47—37425, Japanese Patent Publication No. 48-36894, Japanese Patent Laid-open Applications No. 50—10135, No. 50—117422, No. 50—130441, No. 51-108841, No. 50—120334, No. 52—18315, No. 53—52423 and No. 53—105226.

The ballasted acylamino group (Ball) substituted at the 5-position in the phenyl moiety acts as the "ballast" which can maintained the coupler in a specific layer so as to substantially prevent the said coupler dispersing to any other layer in a multi-layer color photographic element and should, therefore, possess sufficient "bulkiness" for such purposes. Illustrative examples thereof include an arylcarbonylamino group and an alkylcarbonylamino group. In the case of the arylcarbonylamino group, the aromatic ring should have substituent(s) having a $C_5$ to $C_{18}$ alkyl chain. Such substituents having a $C_5$ to $C_{18}$ alkyl chain include, for example, an alkyl group, an alkylcarbonyl- or alrylcarbonyl-oxy group, an alkylcarbonyl- or arylcarbonyl-amino group, a sulfonylamino group, an alkoxycarbonyl group, a carbamoyl group, a sulfamoyl group, an alkylamino group or a dialkylamino group. In the case of the alkylcarbonylamino group, there may be typically mentioned those wherein the aliphatic moiety is a $C_5$ to $C_{18}$ alkyl group, a phenoxyalkyl group or a phenylthioalkyl group. In the case of the phenoxyalkyl or phenylthioalkyl group, the phenoxy moiety may have a substituent having a $C_5$ to $C_{18}$ alkyl chain or the alkyl moiety may have 5 to 18 carbon atoms. As the ballasted alkylcarbonyl- or arylcarbonyl- amino group in the present coupler, Ball is particularly a phenoxyalkyl group or a phenylthioalkyl group. Illustrative examples of the alkylcarbonyl- or arylcarbonyl-amino group ballasted with a phenoxyalkyl group are recited below.

α-(3-Pentadecylphenoxy)butaneamido,
α-(2,4-di-tert-amylphenoxy)hexaneamido,
γ-(2,4-di-tert-amylphenoxy)butaneamido,
α-(2,4-di-tert-amylphenoxy)tetradecaneamido,
α-(4-butylsulfonylaminophenoxy)tetradecaneamido,
α-(4,-acetoxyphenoxy)dodecaneamido,
α-{p-[α-(4-hydroxyphenyl)-α,α-dimethyl]tolyloxy}dodecaneamido,
α-(4-carboxyphenoxy)dodecaneamido,
α-(2-chloro-4-butylsulfonylaminophenoxy)tetradecaneamido,
α-(4-dimethylaminosulfonylaminophenoxy)tetradecaneamido,
α-(3-dodecyloxyphenoxy)butaneamido,
α-(4-dodecyloxyphenoxy)butaneamido,

α-(4-hydroxyphenylthio)dodecaneamido, and
α-(4-acetylaminophenylthio)dodecaneamido.

Preferred cyan couplers of the present invention have the formula [III]

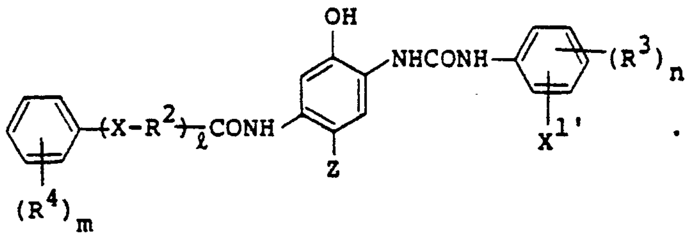 ..... [III]

In the above formula, X is an oxygen atom or a sulfur atom; $R^2$ is a straight or branched alkylene group having 1 to 20 carbon atoms;

$X^{1'}$ is $-COOR^1$, $-SO_2R^1$, $-NO_2$, $-COR^1$, $-SO_2N\begin{smallmatrix}R\\R^1\end{smallmatrix}$, $-CON\begin{smallmatrix}R\\R^1\end{smallmatrix}$, $-SO_2OR^1$ or $-CF_3$ in

which R is a hydrogen atom, an alkyl group (preferably, a $C_1$ to $C_4$ straight or branched alkyl group) or an aryl group (preferably, an unsubstituted or substituted phenyl group);

$R^1$ is an alkyl group (preferably a $C_1$ to $C_4$ straight or branched alkyl group) or an aryl group (preferably an unsubstituted or substituted phenyl group) or R and $R^1$ together form a 5- or 6-membered ring;

$R^3$ is a hydrogen atom, a halogen atom or an optionally substituted alkyl group (preferably $C_1$ to $C_4$ straight or branched alkyl group, particularly preferably, methyl, tert-butyl), alkoxy group (preferably, a $C_1$ to $C_8$ unsubstituted or substituted alkoxy group, particularly preferably methoxy, tert-butoxy, methoxycarbonylmethoxy), aryloxy group (preferably, an unsubstituted or substituted phenoxy group), an unsubstituted or substituted alkylcarbonyloxy group or an arylcarbonyloxy group, alkylcarbonyl or arylcarbonyl group (preferably, a $C_1$ to $C_8$ alkylcarbonyl group, particularly preferably an acetyl group or a pivaloyl group), $R^4$ is a hydrogen atom, a halogen atom (preferably, chlorine or bromine) a hydroxy group, or an optionally substituted alkyl (preferably, a $C_1$ to $C_{20}$ straight or branched alkyl group, particularly preferably methyl, tert-butyl, tert-phentyl, tert-octyl, dodecyl or pentadecyl), aryl (preferably, phenyl), heterocyclic (preferably, an N-containing heterocyclic group), aralkyl (preferably, benzyl or phenethyl), (preferably, a $C_1$ to $C_{20}$ straight or branched alkoxy group, particularly preferably methoxy, ethoxy, tert-butoxy, octyloxy, decyloxy, dodecyloxy), aryloxy (preferably, phenoxy), alkylcarbonyloxy or arylcarbonyloxy group (preferably acetoxy or benzoyloxy), carboxy, alkoxycarbonyl (preferably, a $C_1$ to $C_{20}$ straight or branched, unsubstituted or substituted alkyloxycarbonyl group), aryloxycarbonyl (preferably, an unsubstituted or substituted phenoxycarbonyl group), mercapto, an alkylthio group (preferably, a $C_1$ to $C_{20}$ straight or branched, unsubstituted or substituted alkylthio group), arylthio (preferably, an unsubstituted or substituted phenylthio group), alkylsulfonyl (preferably, a $C_1$—$C_{20}$ straight or branched alkylsulfonyl group), arylsulfonyl (preferably, an unsubstituted or substituted benzenesulfonyl group), alkylcarbonyl or arylcarbonyl (preferably, a $C_1$ to $C_{20}$ straight or branched alkylcarbonyl group or an unsubstituted or substituted benzenesulfonyl group), alkylcarbonyl- or arylcarbonyl-amino group (preferably, a $C_1$ to $C_{20}$ straight or branched alkylcarbon-amido group or an unsubstituted or substituted benzene-carbonamido group), sulfonamido (preferably, a $C_1$ to $C_{20}$ straight or branched, unsubstituted or substituted alkylsulfonamido group or an unsubstituted or substituted benzene-sulfonamido group), carbamoyl (preferably, a $C_1$ to $C_{20}$ straight or branched alkylaminocarbonyl group or an unsubstituted or substituted phenylaminocarbonyl group) or sulfamoyl group (preferably, a $C_1$ to $C_{20}$ straight or branched alkylaminosulfonyl group or an unsubstituted or substituted phenylaminosulfonyl group); Z is as defined in the above formula [I]; n is 0 or an integer from 1 to 3; m is an integer from 1 to 4 and l is O or 1.

Illustrative examples of the coupler, which may be employed in the photosensitive material for color photography according to this invention, are given hereinbelow.

(43)

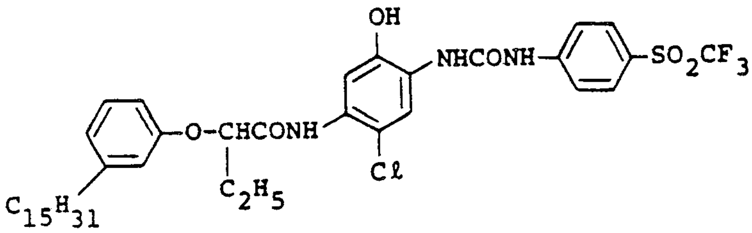

(44)

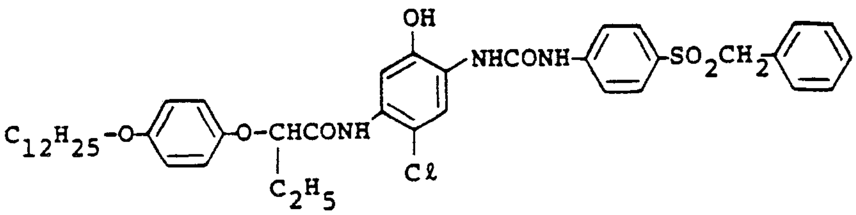

(45)

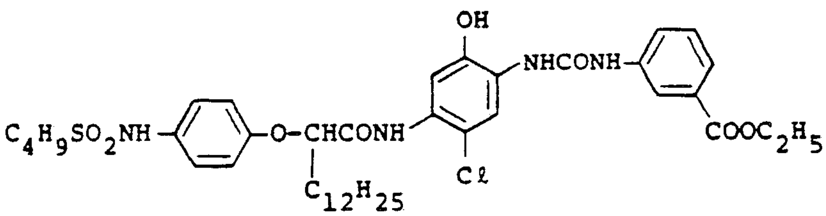

(46)

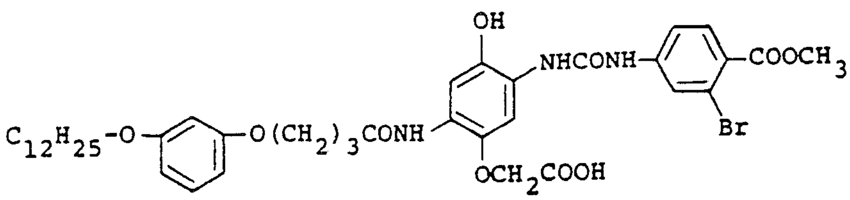

(47)

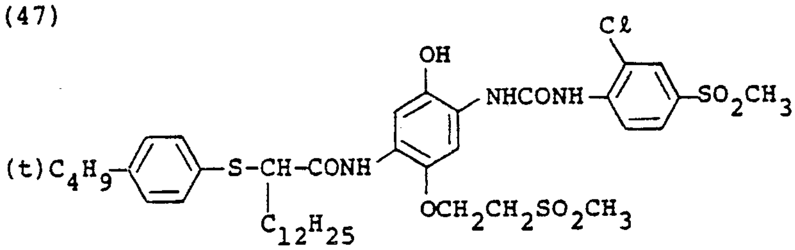

(48)

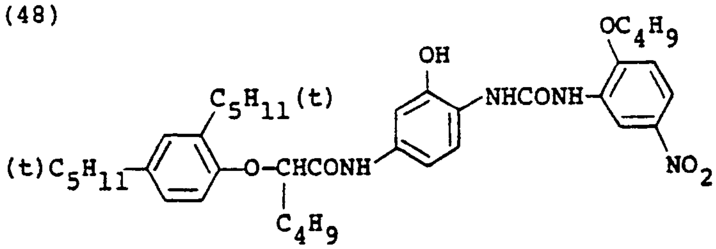

(49)

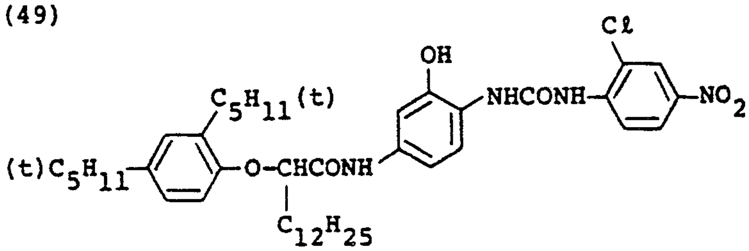

(50)

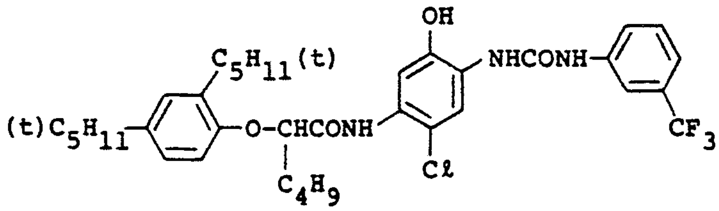

(51)

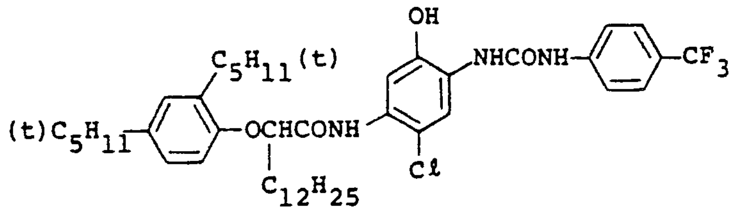

(52)

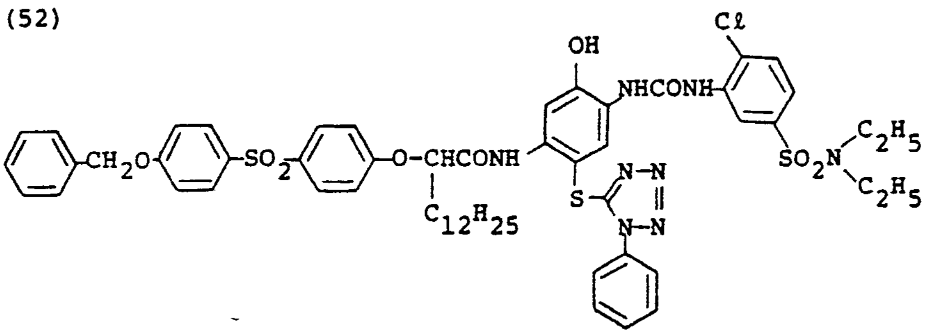

(53)

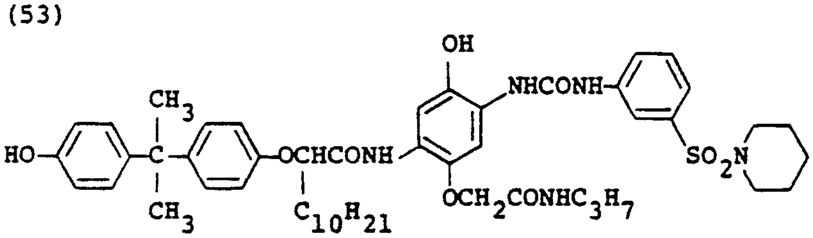

(54)

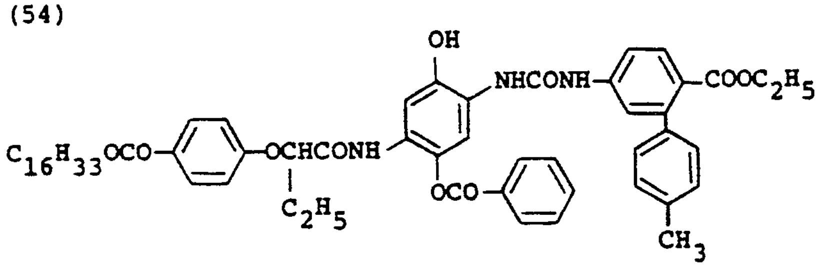

(55)

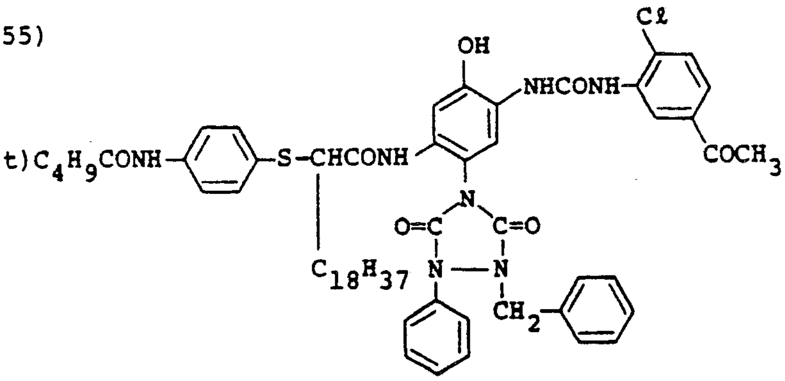

(56)

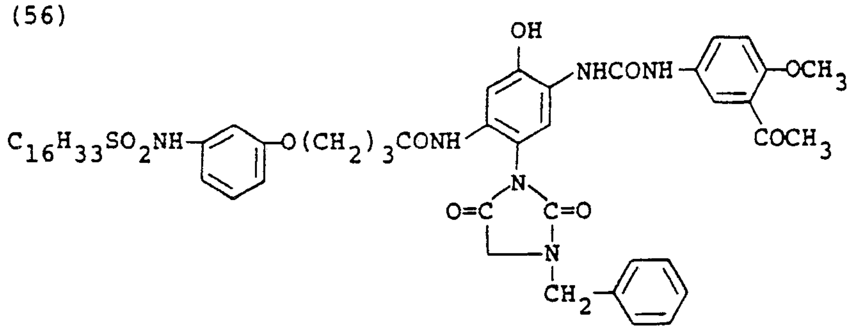

(57)

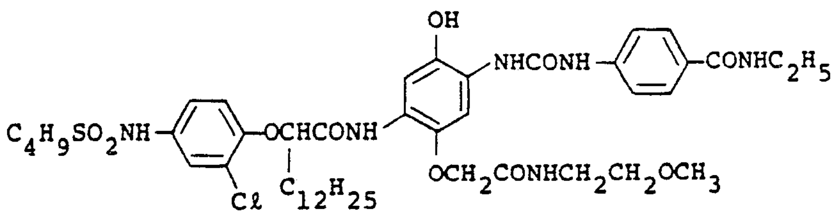

(58)

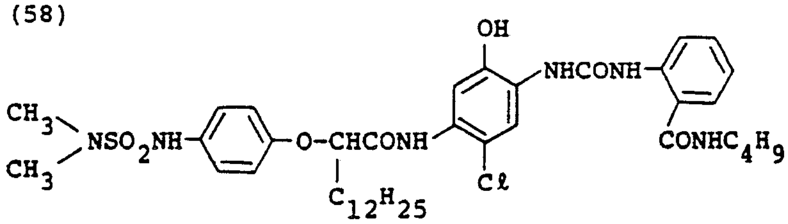

(59)

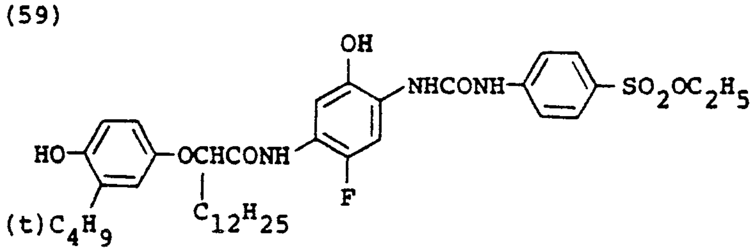

(60)

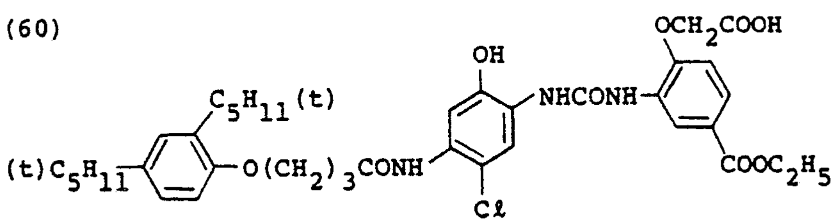

(61)

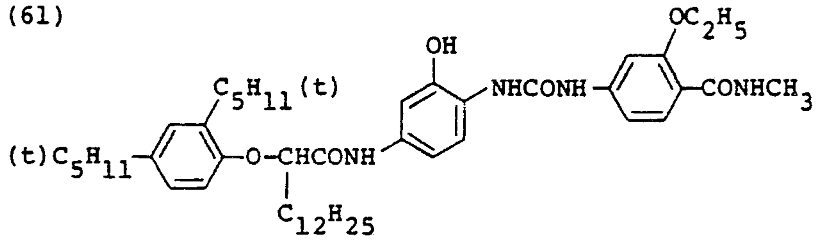

(62)

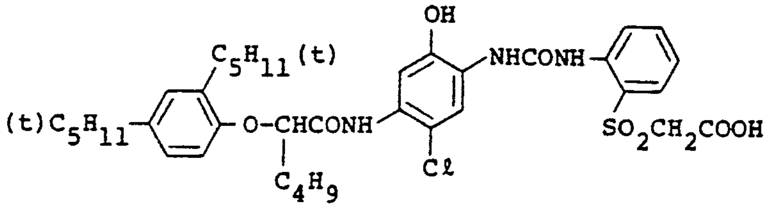

(63)

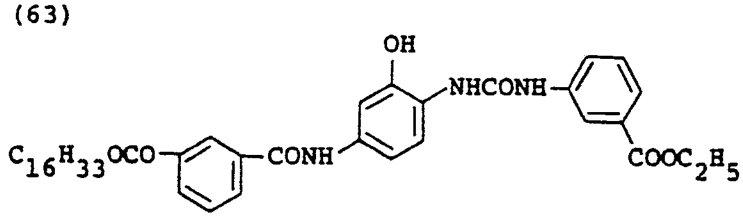

(64)

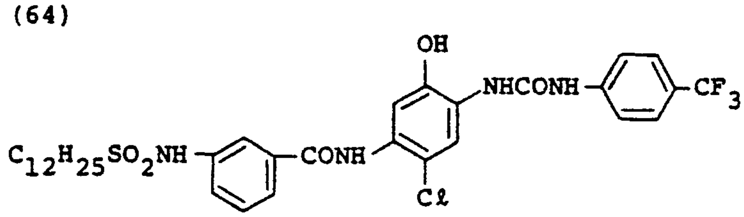

(65)

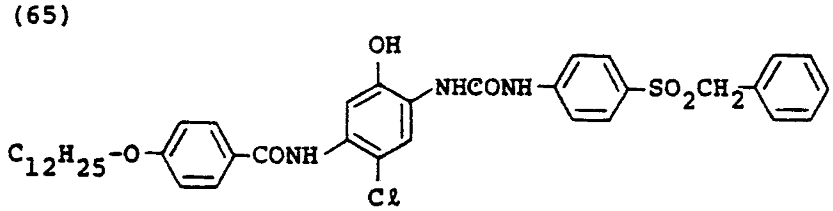

(66)

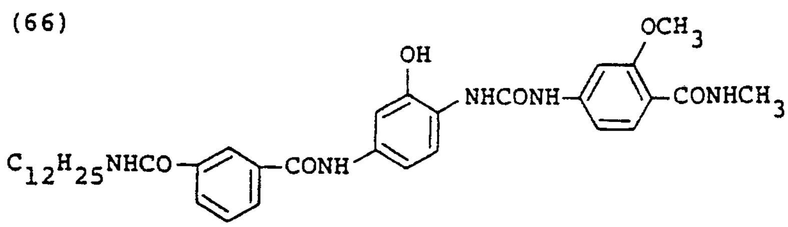

(67)

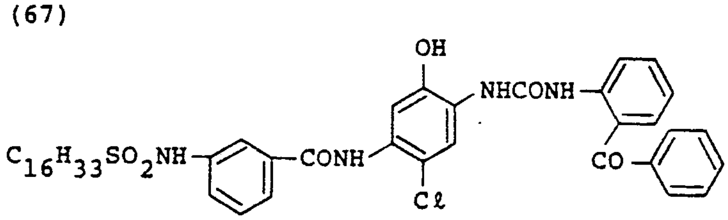

(68)

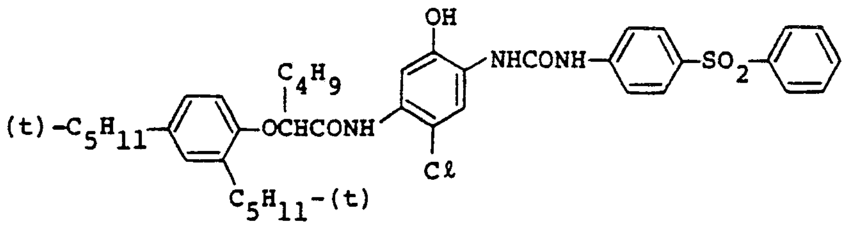

(69)

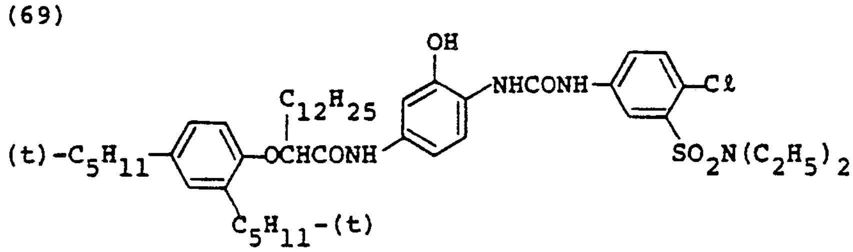

(70)

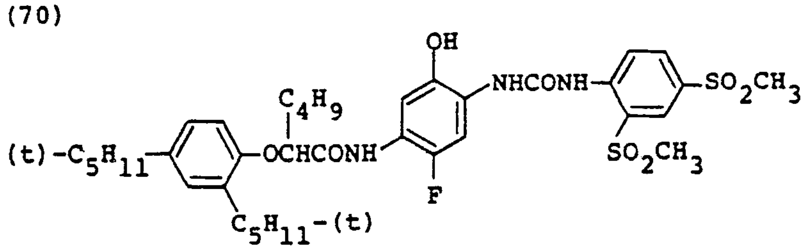

(72)

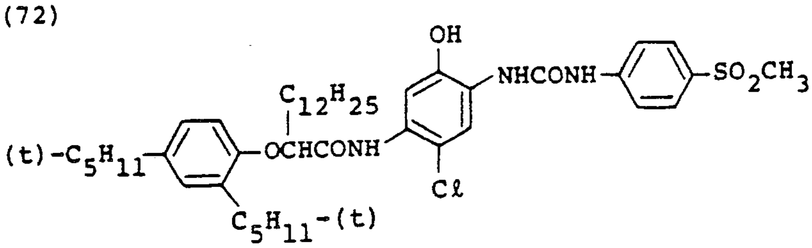

(74)

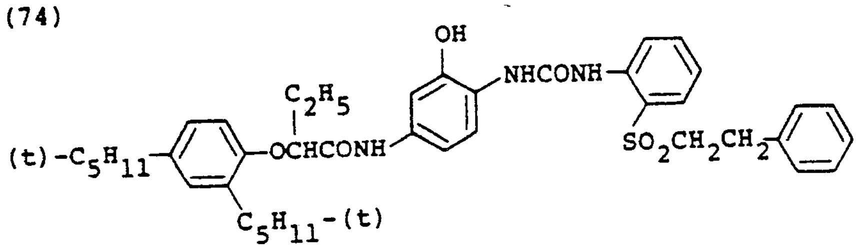

(75)

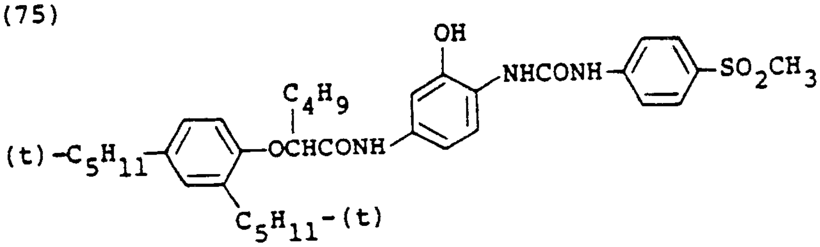

(77)

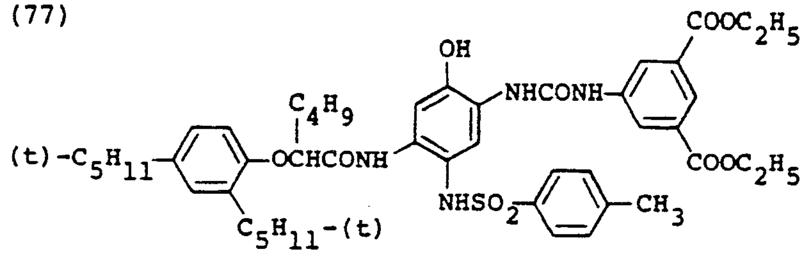

(79)

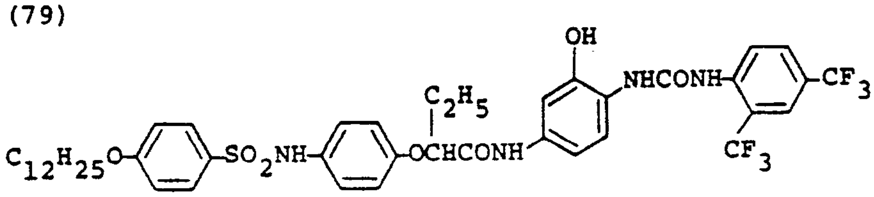

(80)

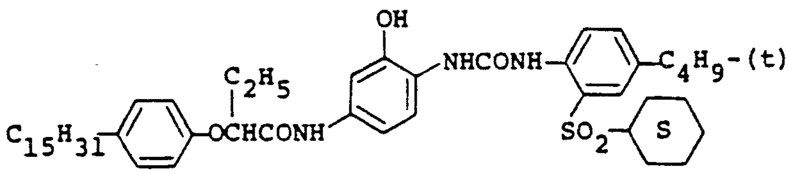

(81)

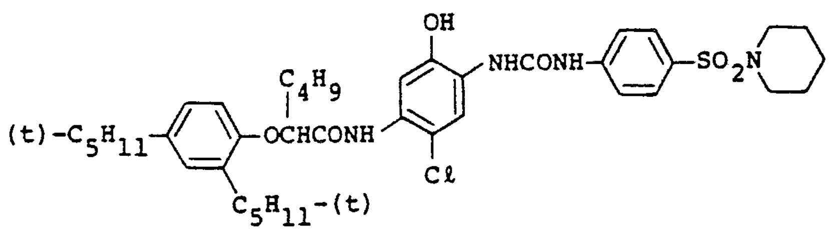

(82)

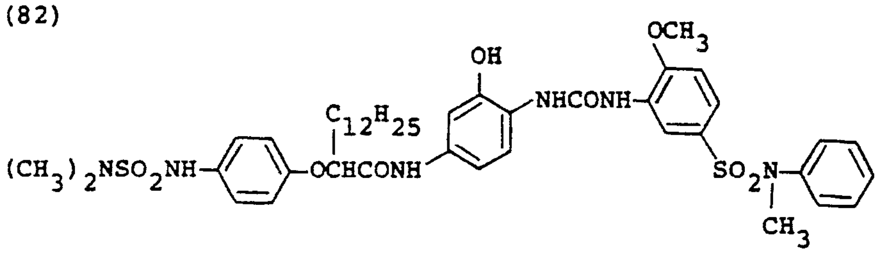

(83)

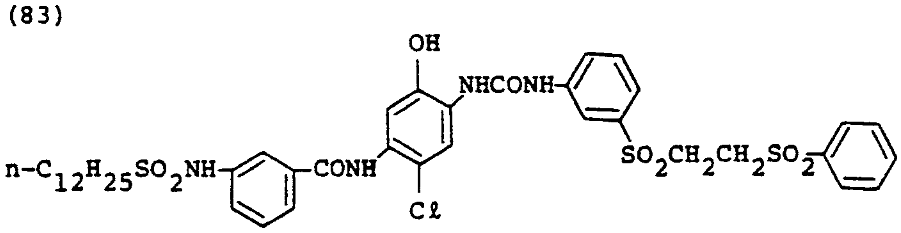

(84)

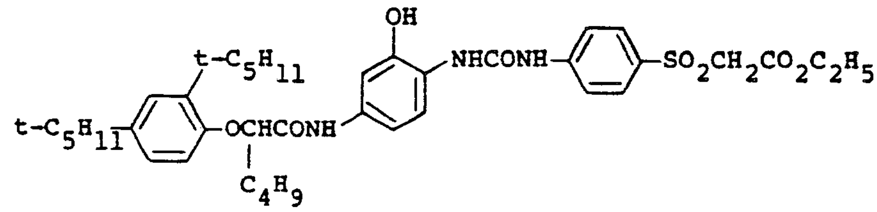

(85)

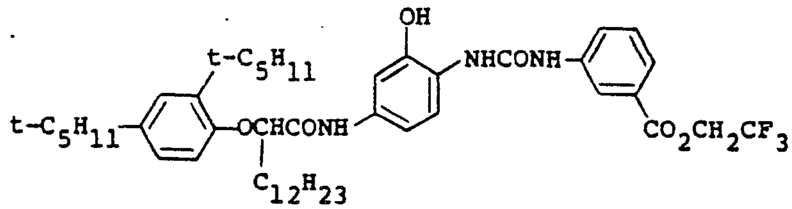

(86)

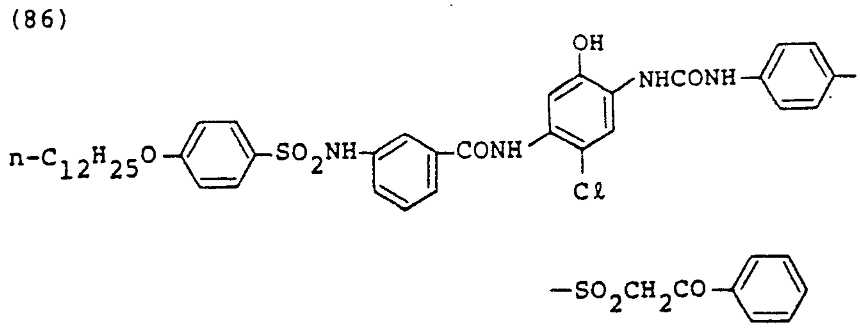

(87)

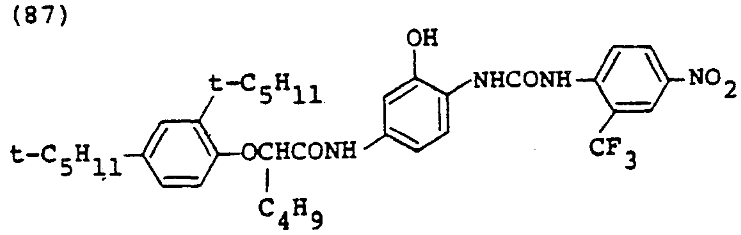

The couplers which may be employed in this invention can be prepared by reacting a substituted p-cyanophenyl isocyanate with a suitable aminophenol, for example, 2-amino-5-nitrophenol or 2-amino-4-chloro-5-nitrophenol to produce a 2-(substituted p-cyanophenyl)ureido compound. Then, the nitro group of the latter compound is reduced to the amino group in a conventional manner and the ballast group is attached to the said amino group to produce the desired coupler.

Representative synthesis examples of the present coupler are given below.

Synthesis Example 1

Coupler No. 45

*Synthesis of 2-(3-ethoxycarbonylphenyl)ureido-4-chloro-5-{α-(4-butylsulfonylamidophenoxy)tetradecane-amido}phenol*

To a suspension of 18.9 g of 2-amino-4-chloro-5-nitrophenol in 200 ml of toluene was added a solution of 21 g of 3-ethoxycarbonylphenyl isocyanate in 100 ml of toluene under stirring at room temperature. The resulting mixture was boiled under reflux for 1 hour and then allowed to cool to room temperature. The crystalline substance thus separated was filtered, washed with methanol and then dried to give 34 g of the product as a pale yellow crystal.

A mixture of 19 g of 2-(3-ethoxycarbonylphenyl)ureido-4-chloro-5-nitrophenol in 600 ml of alcohol was subjected to catalytic reduction using palladium carbon catalyst. After a theoretical volume of hydrogen

was absorbed, the catalyst was filtered off and the filtrate was concentrated under reduced pressure to give 17 g of the crude product.

To a homogeneous solution of 3.5 g of the so obtained 2-(4-ethoxycarbonylphenyl)ureido-4-chloro-5-aminophenol in a mixture of 100 ml of acetonitrile and 0.9 ml of pyridine was added a solution of 4.7 g of α-(4-butylsulfonylamidophenoxy)tetradeconyl chloride in 50 ml of acetonitrile under stirring at room temperature. After completion of the addition, the reaction was continued for further 1 hour, the reaction mixture was added to ice-water, extracted with ethyl acetate, the extract was washed with water, dried over sodium sulfate and concentrated under reduced pressure. The residue was purified by silica gel column chromatography and solidified with hexane to give 3.7 g of the end product as a white solid with mp 146—149°C.

Analysis (%)

| | C | H | N | Cl | S |
|---|---|---|---|---|---|
| Calc'd: | 61.01 | 7.04 | 7.12 | 4.50 | 4.07 |
| Found: | 59.89 | 7.12 | 7.09 | 4.63 | 3.85 |

Synthesis Example 2
Coupler No. 50

*Synthesis of 2-(3-trifluoromethyl)phenylureido-4-chloro-5-{α-(2,4-di-tert-pentylphenoxy)hexane-amido}phenol*

To a suspension of 8.9 g of 2-amino-4-chloro-5-nitrophenol in 200 ml of toluene were added 20.6 g of 3-trifluoromethylphenyl isocyanate under stirring at room temperature. The reaction mixture was boiled under relfux for 3 hours and thereafter allowed to cool to room temperature. The crystalline substance thus separate was filtered, washed with methanol and dried to give 36 g of a pale yellow solid.

A mixture of 18.8 g of the so obtained 2-(3-tri-fluoromethyl)phenylureido-4-chloro-5-nitrophenol in 600 ml of ethanol was subjected to catalytic reduction using palladium-carbon catalyst. After a theoretical volume of hydrogen was absorbed, the catalyst was removed by filtration while hot. The filtrate was concentrated under reduced pressure to give 16 g of crude crystalline substance.

To a mixture of 3.5 g of the so obtained 2-(3-trifluoromethyl)phenylureido-4-chloro-5-aminophenol in a mxiture of 100 ml of acetonitrile with 0.9 ml of pyridine was added a solution of 3.7 g of α-(2,4-di-tert-pentylphenoxy)hexanoyl chloride in 50 ml of acetonitrile under stirring at room temperature. After completion of the addition, the reaction was continued for further 1 hour, added to ice-water and extracted with ethyl acetate. The extract was washed with water, dried over sodium sulfate and then concentrated under reduced pressure to leave an oily substance. The crude substance thus obtained was purified by silica gel column chromatography and solidified with hexane to give 3.6 g of a white solid with mp 151—156°C.

Analysis (%)

| | C | H | N | Cl | F |
|---|---|---|---|---|---|
| Calc'd: | 63.94 | 6.71 | 6.22 | 5.24 | 8.23 |
| Found: | 64.11 | 6.68 | 6.19 | 5.15 | 8.34 |

The cyan dye-forming coupler, which may be employed in this invention, can be used according to the methods and techniques commonly employed for conventional cyan dye-forming couplers. Typically the coupler can be blended with a silver halide emulsion and the emulsion coated onto a base to form a photographic element.

The photographic element may be monochromatic or multicolor one. In the case of a multicolor photographic element, the present cyan dye-forming coupler is usually incorporated into a red sensitive emulsion, but the element will also contain a non-sensitized emulsion or dye-image forming constituent units having photosensitivity to the three primary colors of the spectrum. Each constituent unit may comprise a monoemulsion layer or a multi-emulsion layer which has a photosensitivity to a certain region in spectrum. Element layers including the image-forming constituent layer may be arranged in any optional order as is well known to those skilled in the art. Typical multi-color photographic elements comprise a cyan dye-forming image-forming constituent unit, said unit comprising at least one red sensitive silver halide emulsion layer containing at least one cyan dye-forming coupler (at least one of the couplers being a coupler of this invention) and a yellow dye image-forming constituent unit, said unit comprising at least one blue sensitive silver halide emulsion layer containing at least one magenta dye-forming coupler, both units being carried on a base. The element may further contain additional layers, for example a filter layer, an interlayer, a protective layer and a subbing layer.

The present coupler may be incorporated into an emulsion according to any well-known techniques. For instance, the present coupler alone or in combination with other ingredients may be dissolved in a high boiling organic solvent (boiling point of 175°C or above) such as tricresyl phosphate or dibutyl phthalate or a low boiling organic solvent such as butyl acetate or butyl propionate alone or, if necessary, in combination therewith, the resulting solution is admixed with an aqueous solution of gelatin containing a surface active agent, the resulting mixture is then emulsified by a high speed rotary mixer or a colloid mixer and incorporated with a silver halide to prepare a silver halide emulsion which may be employed in this

invention. The coupler of this invention is suitably employed in a range of usually about 0.07—0.7 mole, preferably 0.1—0.4 mole, per mole of silver halide of the silver halide emulsion.

As the silver halide which may be employed in the present silver halide emulsion, there may be used any silver halide commonly employed for a silver halide emulsion such as silver bromide, silver chloride, silver iodobromide, silver chlorobromide or silver chloroiodobromide.

A silver halide emulsion for the present invention may be prepared by various conventional methods such as the method disclosed, for example, in Japanese Patent Publication No. 46—7772: namely, a method for preparing a so-called conversion emulsion wherein a silver salt grain emulsion is formed, the said grain comprising at least partly a silver salt having a higher solubility than that of silver bromide, and then at least part of the said silver salt is converted to silver bromide or silver iodobromide, or a method for preparing the so-called Lippmann emulsion comprising silver halide grains having an average grain size of not more than 0.1 μ.

Moreover, the silver halide emulsion may be chemically sensitized with a sulfur sensitizer such as allythiocarbamide, thiourea or cystine, an active or inactive selenium sensistizer, a reduction sensitizer such as a stannous salt, a polyamine, a noble metal sensitizer such as a gold sensitizer, typically potassium aurithiocyanate, potassium chloroaurate or 2-aurosulfobenzothiazole methyl chloride, or a water-soluble ruthenium, rhodium or irridium, for example, salt sensitizer, typically ammonium chloropalladate, potassium chloroplatinate or sodium chloropalladate alone or in any combination.

The silver halide emulsion used herein may also contain a wide variety of well-known photographic additives, for example, those disclosed in "Research Disclosure", 1978, December, item 17643.

The silver halide emulsion may be spectrally sensitized using any suitable sensitizing dyes in order to afford photosensitivity to the sensitive wave length region required for a red sensistive emulsion. One or more spectrally sensitizing dyes may be used, for example those cyanine dyes, merocyanine dyes or complex cyanine dyes disclosed in U.S. Patents No. 2,269, 234, No. 2,270,378, No. 2,442,710, No. 2,454,629, No. 2,776,280.

The color developing solution which may be employed in this invention preferably contains as a main ingredient an aromatic primary amine type color developing agent. Illustrative examples of such color developing agents are typically of the p-phenylenediamine type; for example; diethyl-p- phenylenediamine hydrochloride, monomethyl-p-phenylenediamine hydrochloride, dimethyl-p-phenylenediamine hydrochloride, 2-amino-5-diethylaminotoluene hydrochloride, 2-amino-5-(N-ethyl-N-dodecylamino)toluene, 2-amino-5-(N-ethyl-N-β-methane-sulfonamidoethyl)aminotoluene sulfate, 4-(N-ethyl-N-β-methanesulfonamidoethylamino)aniline, 2-amino-5-(N-ethyl-N-β-methoxyethyl)aminotoluene and 4-(N-ethyl-N-β-hydroxyethylamino)aniline.

After development, conventional steps of bleaching for removal of silver or silver halide, fixing or bleach-fixing, washing and drying may be applied.

The following Examples are givenn to illustrate this invention further.

Example 1

0.03 mole of each of the couplers indicated in the following Table 1 and the following control couplers A, B and C was added separately to a mixture of the same weight of dibutyl phthalate and 3 times the volume of ethyl acetate and the mixture was heated to 60°C to form a complete solution. The solution was added to an aqueous solution of "Alkanol B" (alkylnaphthalene sulfonate, Registered Trade Mark of E.I. DuPont) and gelatin, the resulting mixture was emulsified by the colloid mill to prepare coupler dispersions of each coupler. Then, each coupler dispersion was added to a silver chlorobromide emulsion (20 mole % silver bromide; containing 0.1 mole silver) and the mixture was coated onto a polyethylene laminated paper and then dried to prepare 6 silver halide photosensitive materials for color photography having a stable coated film (Samples No. 31 to No. 36).

Control coupler A:

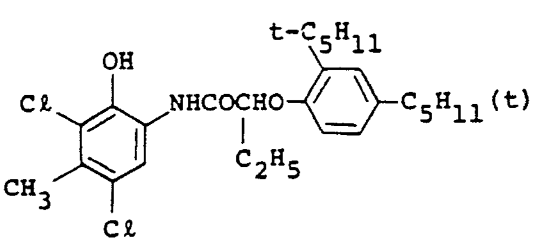

Control coupler B:

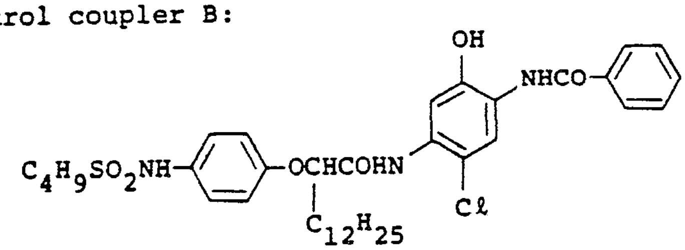

Control coupler C:

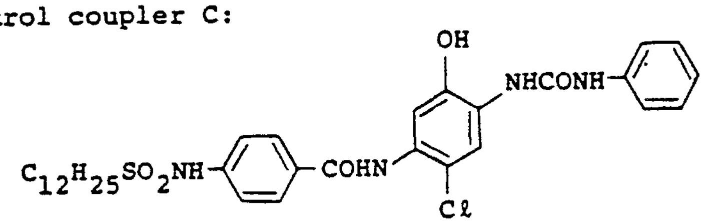

Each sample was subjected to wedge exposure according to a conventional method and then treated as stated hereunder. However, the color developing step was effected with two sorts of color developing compositions, namely color development (1) with benzylalcohol and color development (2) without benzyl alcohol.

(Treatment)

| Treatment step (30°C) | Treatment time |
|---|---|
| Color development | 3 min. 30 sec. |
| Bleach-fixing | 1 min. 30 sec. |
| Water washing | 2 min. |

Formulations for each step are given below.

(Colour developing solution, composition 1)

| | |
|---|---|
| 4-Amino-3-methyl-N-ethyl-N-(β-methanesulfonamindoethyl)-aniline sulfate | 5.0 g |
| Benzyl alcohol | 15.0 ml |
| Sodium hexamethaphosphate | 2.5 g |
| Anhydous sodium sulfite | 1.85 g |
| Sodium bromide | 1.4 g |
| Potassium bromide | 0.5 g |
| Borax | 39.1 g |

Water to make up 1 l
Adjusted to pH 10.30 with NaOH

(Colour developing solution, composition 2)

The same formulation as in the above composition 1 except that the benzyl alcohol was omitted.

(Bleach-fixing solution composition)

| | |
|---|---|
| Ethylenediaminetetraacetate iron ammonium complex | 50 g |
| Ammonium sulfite (40% aqueous solution) | 50 ml |
| Ammonium thiosulfate (70% aqueous solution) | 140 ml |
| Aqueous ammonia (28% solution) | 20 ml |
| Ethylenediaminetetraacetic acid | 4 g |
| Water to make up 1 l | |

Each sample was assessed for its photographic characteristics. The results are summarized in Table 1, wherein relative sensitivity values are represented in terms of the maximum sensitivity value when treated with the color developing solution 1 taken as 100.

As can be seen from Table 1, the samples prepared from the present couplers show a good sensitivity and maximum density, irrespective of the presence or absence of benzyl alcohol.

In the Table, light fastness is represented in terms of the residual density of each image after exposure to a xenon fadeometer over 300 hours, the density before exposure being taken as 100; moisture fastness is represented in terms of the residual density after storage under a relative humidity of 70% over 3 weeks, the density before testing being taken as 100; and heat fastness is represented in terms of the residual density after storage at 77°C over 3 weeks, the density before testing being taken as 100, provided that the initial density is 1.0.

As is apparent from the Table, the control coupler A possesses excellent light fastness, but not so good heat and moisture fastness, while control couplers B and C possess excellent heat and moisture fastness but not so good light fastness upon color development 2. In contrast, the present couplers Nos. 44, 45 and 50 are clearly excellent in every respect.

TABLE 1

| Sample No. | Coupler applied | Colour development 1 | | | Colour development 2 | | |
|---|---|---|---|---|---|---|---|
| | | light fastness | heat fastness | moisture fastness | light fastness | heat fastness | moisture fastness |
| 31 | No. 44 | 86 | 98 | 96 | 87 | 98 | 99 |
| 32 | No. 45 | 85 | 97 | 97 | 86 | 100 | 100 |
| 33 | No. 50 | 85 | 98 | 97 | 85 | 97 | 96 |
| 34 | Control coupler A | 84 | 47 | 63 | 85 | 46 | 63 |
| 35 | Control coupler B | 60 | 94 | 92 | 56 | 95 | 92 |
| 36 | Control coupler C | 57 | 93 | 91 | 50 | 94 | 90 |

Example 2

0.01 mole of each of the present couplers indicated in the following Table 2 and the above control couplers A and B and the following control coupler D was added (separately) to a mixture of the same weight of tricresyl phosphate and 3 times the volume of ethyl acetate and the resulting mixture was heated to 60°C. to form a complete solution. The solution was added to an aqueous solution of "Alkanol B" and gelatin, the resulting mixture was emulsified by a colloid mill to prepare coupler dispersons of each coupler.

Then, each coupler disperson was added to a silver iodobromide emulsion (6 mole % silver iodide, contianing 0.1 mole silver) and the mixture was coated onto a cellulose acetate film base and then dried to

prepare 6 silver halide photosensitive materials for color photography having a stable coated film (Samples No. 37 to No. 42).

Control coupler D:

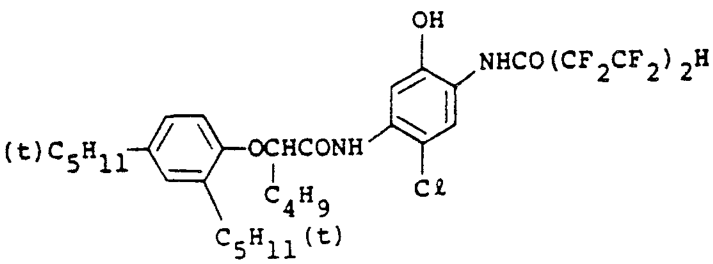

Each sample was subjected to wedge exposure according to a conventional method and then treated as stated hereunder.

(Treatment)

| Treatment step (33°C) | Treatment time |
|---|---|
| Colour development | 3 min. 15 sec. |
| Bleach | 6 min. 30 sec. |
| Water washing | 3 min. 15 sec. |
| Fixing | 6 min. 30 sec. |
| Water washing | 3 min. 15 sec. |
| Stabilization | 1 min. 30 sec. |

(Colour developing solution, composition)

| | |
|---|---|
| 4-Amino-3-methyl-N-ethyl-N-(β-hydroxyethyl)aniline sulfate | 4.8 g |
| Anhydrous sodium sulfate | 0.14 g |
| Hydroxylamine 1/8 sulfate | 1.98 g |
| Sulfuric acid | 0.74 g |
| Anhydrous potassium carbonate | 28.85 g |
| Anhydrous potassium hydrogencarbonate | 3.46 g |
| Anhydrous potassium sulfite | 5.10 g |
| Potassium bromide | 1.16 g |
| Sodium chloride | 0.14 g |
| Nitriloacetic acid trisodium salt | 1.20 g |
| Potassium hydroxide | 1.48 g |
| Water to make up 1 l. | |

(Bleaching solution, composition)

| | |
|---|---|
| Ethylenediaminetetraacetato iron ammonium complex | 100 g |
| Ethylenediaminetetraacetato di-ammonium salt | 10 g |
| Ammonium bromide | 150 g |
| Glacial acetic acid | 10 ml |

Water to make up 1 l.
Adjusted to pH 6.0 with aqueous ammonia

(Fixer, composition)

| | |
|---|---|
| Ammonium thiosulfate | 175.0 g |
| Anhydrous sodium sulfite | 8.6 g |
| Sodium metasulfite | 2.3 g |

Water to make up 1 l.
Adjusted to pH 6.0 with acetic acid

(Stabilizer, composition)

| | |
|---|---|
| Formalin (37% aqueous solution) | 1.5 ml |
| Konidax (manufactured by Konishiroku Photo Ind. Co., Ltd.) | 7.5 ml |

Water to make up 1 l.

The produced cyan color images were assessed for photographic characterisitcs. The results are summarized in Table 2.

TABLE 2

| Sample No. | Coupler applied | Relative sensitivity | Maximum density |
|---|---|---|---|
| 37 | No. 50 | 97 | 2.18 |
| 38 | No. 57 | 100 | 2.22 |
| 39 | No. 64 | 100 | 2.20 |
| 40 | Control coupler A | 100 | 1.67 |
| 41 | Control coupler B | 92 | 1.61 |
| 42 | Control coupler D | 81 | 1.50 |

As apparent from the above Table 2, the samples using the couplers of this invention possess excellent sensitivity and color development. Also, the samples using the couplers of this invention have been found to produce a favourable dye image in color reproduction in the green region, showing an absorption maximum in a long wave length range of the red region and sharpness in a short wave length range.

Example 3

The samples No. 31 to No. 33 prepared in the above Example 1 were subjected to wedge exposure and then developed with the composition 1 in Example 1, except that a developing treatment was carried out

with a bleach-fixing solution having the following composition to study discoloration of the cyan dye with an exhausted bleach-fixing solution.

(Bleach-fixing solution, composition)

| | |
|---|---|
| Ethylenediaminetetraacetato iron ammonium complex | 50 g |
| Ammonium sulfite (40% solution) | 50 ml |
| Ammonium thiosulfate (70% solution) | 140 ml |
| Aqueous ammonia (28% solution) | 20 ml |
| Ethylenediaminetetraacetic acid | 4 g |
| Hydrosulfite | 5 g |
| Water to make up 1 l. | |

The sample thus treated was assessed for reflection density of the cyan dye. The results are summarized in Table 3. Dye residual rate at the maximum density is calculated from the following equation.

$$\text{Dye residual rate} = \frac{\text{Treatment with fresh bleach-fixing solution}}{\text{Treatment with exhausted bleach-fixing solution}} \times 100$$

In the above Table 1, light fastness, moisture fastness and heat fastness are represented in the same manner as in the above Table 2.

The present couplers No. 44, No. 45 and No. 50 have been found to show excellent properties in every respect.

TABLE 3

| Coupler applied | Fresh BF* treatment | Exhausted BF treatment | Dye residual rate |
|---|---|---|---|
| No. 45 | 2.18 | 2.18 | 100 |
| No. 51 | 2.22 | 2.20 | 99 |
| No. 65 | 2.18 | 2.18 | 100 |
| Control coupler A | 2.16 | 1.38 | 64 |
| Control coupler B | 1.90 | 1.86 | 98 |
| Control coupler C | 1.82 | 1.75 | 96 |

*BF = Bleach-Fixing Solution

It can be seen from the above Table 3 that the samples using the present couplers show less discoloration of cyan dye when treated with exhausted bleach-fixing solution.

Example 4

Couplers of the present invention, the above-mentioned Control coupler D and the below-mentioned Control couplers E and F were prepared, exposed and then developed in the same manner as in Example 2 to obtain Samples (Sample Nos. 43 to 49). The thus obtained Samples were tested for light fastness, heat fastness and moisture fastness of the cyan dye image. The results are shown in Table 4.

TABLE 4

| Sample No. | Coupler applied | light fastness | heat fastness | moisture fastness |
|---|---|---|---|---|
| 43 | No. 49 | 83 | 96 | 97 |
| 44 | No. 59 | 82 | 95 | 95 |
| 45 | No. 67 | 86 | 99 | 98 |
| 46 | No. 81 | 88 | 96 | 98 |
| 47 | Control coupler D | 56 | 82 | 88 |
| 48 | Control coupler E | 68 | 79 | 85 |
| 49 | Control coupler F | 82 | 90 | 94 |

Control coupler E:

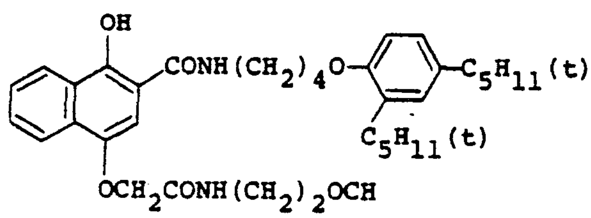

Control coupler F:

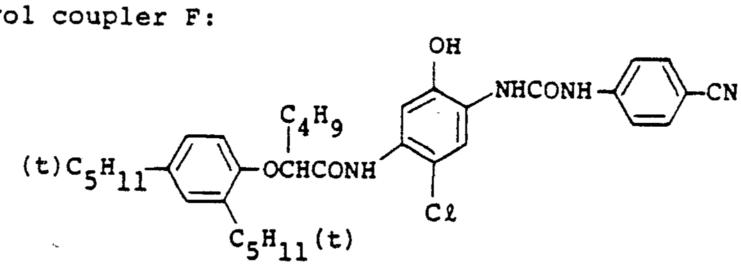

As can be seen from Table 4, the Samples obtained by using the cyan couplers according to the present invention exhibit excellent properties in respect of light fastness, heat fastness and moisture fastness.

Example 5

Samples were obtained in the same manner as in Example 1 except that the couplers shown in Table 5 were used, and their photographic properties were measured according to the same procedures as in Example 1. The results are shown in Table 5.

TABLE 5

| Sample No. | Coupler applied | Color development 1 | | Color development 2 | |
|---|---|---|---|---|---|
| | | Relative sensitivity | Maximum density | Relative sensitvity | Maximum density |
| 53 | 44 | 100 | 2.18 | 72 | 1.80 |
| 54 | 45 | 100 | 2.20 | 74 | 1.83 |
| 55 | 50 | 99 | 2.19 | 71 | 1.80 |

**Claims**

1. A silver halide photosensitive material suitable for color photography which comprises a phenol type cyan coupler having the formula

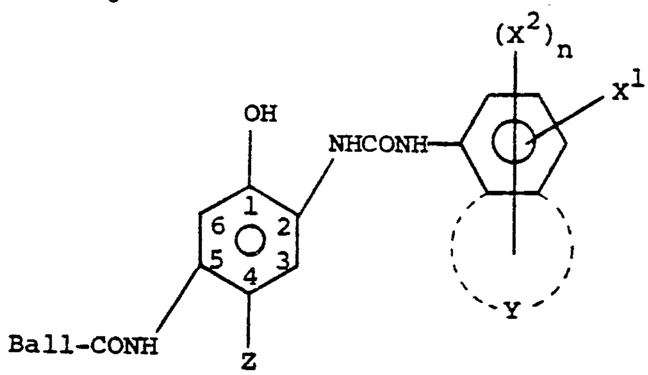

[I]

wherein

$X^1$ is $—COOR^1$, $—COR^1$, $—SO_2OR^1$, $—SO_2R^1$, 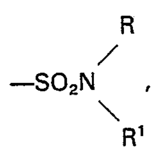 , 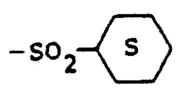 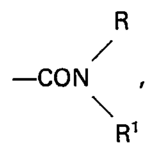 , $—NO_2$

or $—CF_3$, in which

$R^1$ is an optionally substituted alkyl or aryl group and R is a hydrogen atom, or an optionally substituted alkyl, or aryl group; or R and $R^1$ together for a 5 or 6 membered ring;
$X^2$ is a halogen atom, a hydroxy group, a nitro group to an optionally substituted alkyl, alkylcarbonyloxy, arylcarbonyloxy, alkoxy, aryloxy, alkylcarbonyl or arylcarbonyl group;
Y, when present, is a group of non-metallic atoms which completes a 5- or 6-membered condensed ring;
Z is a hydrogen atom or an atom or group removable upon coupling with an oxidation product of a color developing agent;
Ball is a ballast group; and
n is 0 or an integer from 1 to 4 such that when n is 2 or more, each $X^2$ may be the same or different.

2. A silver halide photosensitive material according to Claim 1, wherein said phenol type cyan coupler has the formula:

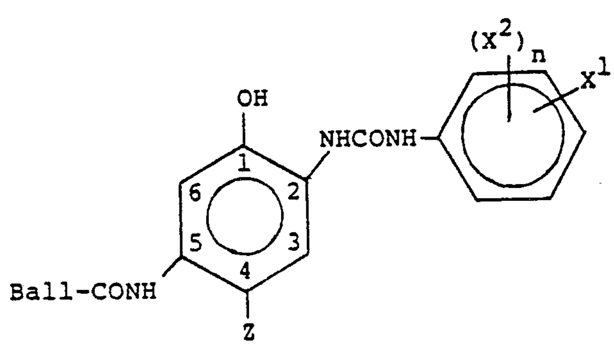

wherein $X^1$, $X^2$, z, Ball and n are as defined in Claim 1.

3. A silver halide photosensitive material according to Claim 2, wherein the Ball—CONH— group is an alkylcarbonylamino or arylcarbonylamino group.

4. A silver halide photosensitive material according to any one of Claims 1 to 3, wherein n is zero.

5. A silver halide photosensitive material according to Claim 1, wherein said phenol type cyan coupler has the formula:

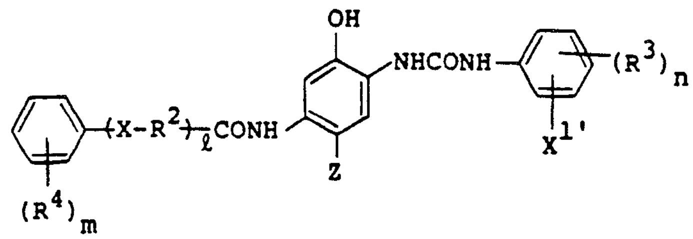

wherein

X is an oxygen atom or a sulfur atom;

$R^2$ is a straight or branched $C_1$ to $C_{20}$ alkylene group;

$R^3$ is a hydrogen atom, a halogen atom or an optionally substituted alkyl, alkylcarbonyloxy, arylcarbonyloxy, alkoxy, aryloxy, alkylcarbonyl or arylcarbonyl group; $R^4$ is a hydrogen or halogen atom, a hydroxy group or an optionally substituted alkyl, aryl, heterocyclic, aralkyl, alkoxy, aryloxy, alkylcarbonyloxy, arylcarbonyloxy, carboxy, alkoxycarbonyl, aryloxycarbonyl, mercapto, alkylthio, arlythio, alkylsulfonyl, arylsulfonyl, alkylcarbonyl, arylcarbonyl, alkylcarbonylamino, arylcarbonylamino, sulfonamido, carbamoyl or sulfamoyl group;

$X^1$ is $-COOR^1$, $-COR^1$, $-SO_2OR^1$, $-SO_2R^1$, $-SO_2N\begin{matrix}/R\\ \backslash R^1\end{matrix}$, $-CON\begin{matrix}/R\\ \backslash R^1\end{matrix}$, $-NO_2$ or $-CF_3$

in which R is a hydrogen atom, an alkyl or an aryl group and $R^1$ is an alkyl group or an aryl group;

n is 0 or an integer from 1 to 3;

m is an integer 1 to 4;

l is 0 or 1, and z is as defined in Claim 1.

6. A silver halide photosensitive material according to Claim 1, 3 or 4, wherein $X^1$ represents $-COOR^1$, $-COR^1$, $-SO_2OR^1$, $-SO_2R^1$, $-CON\begin{matrix}/R\\ \backslash R^1\end{matrix}$, $-NO_2$ or $CF_3$ in which $R^1$ and R are as defined in Claim 1.

7. A silver halide photosensitive material according to any one of Claims 1 to 3 wherein $X^1$ is $-SO_2R^1$ in which $R^1$ is as defined in Claim 1.

8. A silver halide photosensitive material according to Claim 1 wherein $R^1$ is an alkyl or aryl group, R is a hydrogen atom or an alkyl or aryl group and Z is a hydrogen atom or a group removable upon coupling with an oxidation product of a color developing agent.

9. A photosensitive silver halide emulsion containing a phenol type cyan coupler as defined in any one of Claims 1 to 9.

10. A photosensitive element which comprises an emulsion as claimed in Claiim 9 coated on a base.

11. A phenol type cyan coupler as defined in any one of claims 1 to 8.

**Patentansprüche**

1. Lichtempfindliches farbphotographisches Silverhalogenid-Aufzeichnungsmaterial mit einem Blaugrünkuppler von Phenoltyp der Formel:

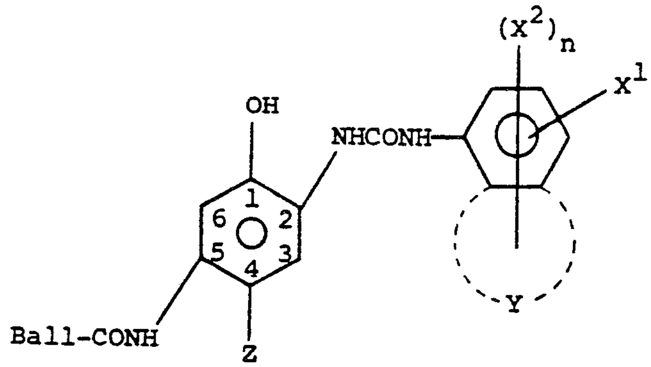

worin bedeuten:

$X^1$ is $—COOR^1$, $—COR^1$, $—SO_2OR^1$, $—SO_2R^1$, 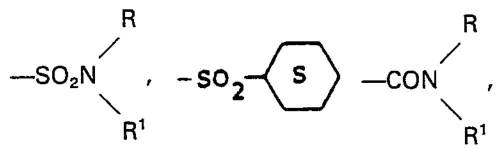 , $—NO_2$

oder $—CF_3$, worin $R^1$ für

eine gegebenenfalls substituierte Alkyl- oder Aryl-gruppe steht, R ein Wasserstoffatom oder eine gegebenenfalls substituierte Alkyl- oder Arylgruppe darstellt oder R und $R^1$ zusammen einen 5- oder 6-gliedrigen Ring bilden;
$X^2$ ein Halogenatom oder eine Hydroxy-, Nitro- oder gegebenenfalls substituierte Alkyl-, Alkylcarbonyloxy-, Arylcarbonyloxy-, Alkoxy-, Aryloxy-, Alkylcarbonyl- oder Arylcarbonylgruppe;
Y, falls vorhanden, eine Gruppe nicht-metallischer Atome, die einen 5- oder 6-gliedrigen ankondensierten Ring vervollständigen;
Z ein Wasserstoffatom oder ein Atom oder eine Gruppe, das oder die bei der Kupplung mit einem Oxidations- produkt einer Farbentwicklerverbindung enfernbar ist;
Ball eine Ballastgruppe und
n 0 oder eine ganze Zahl von 1 bis 4, wobei gilt, daß im Falle, daß n = 2 oder mehr, die einzelnen Reste $X^2$ gleich oder verschieden sein können.

2. Lichtempfindliches Silberhalogenid-Aufzeichnungs- material nach Anspruch 1, dadurch gekennzeichnet, daß der Blaugrünkuppler vom Phenoltyp der Formel:

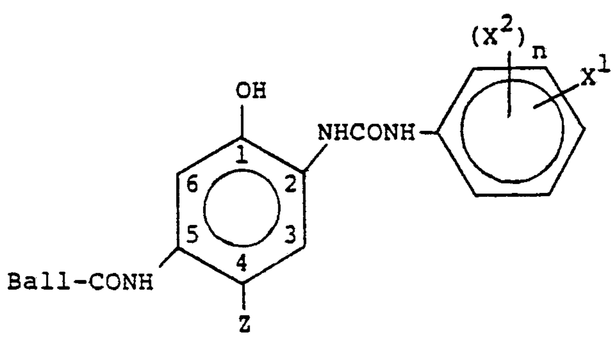

worin $X^1$, $X^2$, z, Ball und n die in Anspruch 1 angegebene Bedeutung bestizen, entspricht.

3. Lichtempfindliches Silberhalogenid-Aufzeichnungsmaterial nach Anspruch 2, dadurch gekennzeichnet, daß die Ball-CONH—Gruppe aus einer Alkylcarbonylamino-oder Arylcarbonylaminogruppe besteht.

4. Lichtempfindliches Silberhalogenid-Aufzeichnungsmaterial nach einem der Ansprüche 1 bis 3, dadurch gekennzeichnet, daß n = 0.

5. Lichtempfindliches Silberhalogenid-Aufzeichnungsmaterial nach Anspruch 1, dadurch gekennzeichnet, daß der Blaugrünkuppler vom Phenoltyp der Formel:

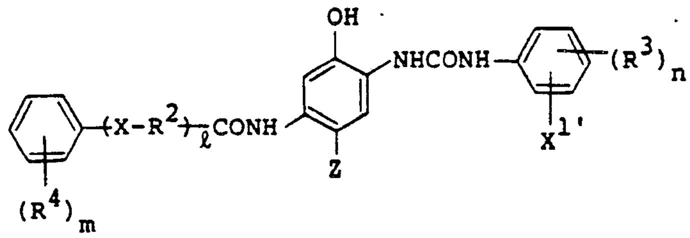

worin bedeuten:
X ein Sauerstoff- oder Schwefelatom;
$R^2$ eine gerad- oder verzweigtkettige $C_1$- bis $C_{20}$- Alkylengruppe;
$R^3$ ein Wasserstoff- oder Halogenatom oder eine gegebenenfalls substituierte Alkyl-, Alkylcarbonyloxy-, Arylcarbonyloxy-, Alkoxy-, Aryloxy-, Alkylcarbonyl- oder Arylcarbonylgruppe;
$R^4$ ein Wasserstoff- oder Halogenatom, eine Hydroxy- gruppe oder eine gegebenenfalls substituierte Alkyl-, Aryl-, heterocyclische, Aralkyl-, Alkoxy-, Aryloxy-, Alkylcarbonyloxy-, Arylcarbonyloxy-, Carboxy-, Alkoxycarbonyl-, Aryloxycarbonyl-, Mercapto-, Alkylthio-, Arylthio-, Alkylsulfonyl-, Arylsulfonyl-, Alkylcarbonyl-, Arylcarbonyl-, Alkylcarbonylamino-, Arylcarbonylamino-, Sulfonamido-, Carbamoyl- oder Sulfamoylgruppe;

$X^1$ $-COOR^1$, $-COR^1$, $-SO_2OR^1$, $-SO_2R^1$, $-SO_2N{<}^{R}_{R^1}$, $-CON{<}^{R}_{R^1}$, $-NO_2$ oder $-CF_3$, worin R für ein Wasserstoffatom oder eine Alkyl- oder Arylgruppe steht und $R^1$ eine Alkyl- oder Arylgruppe darstellt;
n 0 oder eine ganze Zahl 1 bis 3;
m eine ganze Zahl von 1 bis 4 und
l = 0 oder 1 und
worin z die in Anspruch 1 angegebene Bedeutung besitzt,
entspricht.

6. Lichtempfindliches Silberhalogenid-Aufzeichnungsmaterial nach Ansprüchen 1, 3 oder 4, dadurch gekennzeichnet, daß

$X^1$ für $-COOR^1$, $-COR^1$, $-SO_2OR^1$, $-SO_2R^1$, $-CON{<}^{R}_{R^1}$, $-NO_2$ oder $-CF_3$

mit $R^1$ und R gleich der in Anspruch 1 angegebenen Bedeutung steht.

7. Lichtempfindliches Silberhalogenid-Aufzeichnungsmaterial nach einem der Ansprüche 1 bis 3, dadurch gekennzeichnet, daß $X^1$ für $-SO_2R^1$ mit $R^1$ gleich der in Anspruch 1 angegebenen Bedeutung steht.

8. Lichtempfindliches Silberhalogenid-Aufzeichnungsmaterial nach Anspruch 1, dadurch gekennzeichnet, daß $R^1$ für eine Alkyl- oder Arylgruppe steht; R ein Wasserstoffatom oder eine Alkyl- oder Arylgruppe darstellt und Z ein Wasserstoffatom oder eine bei der Kupplung mit einem Oxidationsprodukt einer Farbentwicklerverbindung entfernbare Grupe bedeutet.

9. Lichtempfindliche Silberhalogenidemulsion mit einem Blaugrünkuppler vom Phenoltyp entsprechend der Definition nach einem der Ansprüche 1 bis 8.

10. Lichtempfindliches Aufzeichnungsmaterial, bei welchem auf einen Schichtträger eine Emulsion nach Anspruch 9 aufgetragen ist.

11. Blaugrünkuppler vom Phenoltyp gemäß der Definition nach einem der Ansprüche 1 bis 8.

**Revendications**

1. Matériau photosensible à base d'halogénure d'argent pour photographie couleur, constitué par un couplant bleu-vert du type phénolique réspondant à la formule

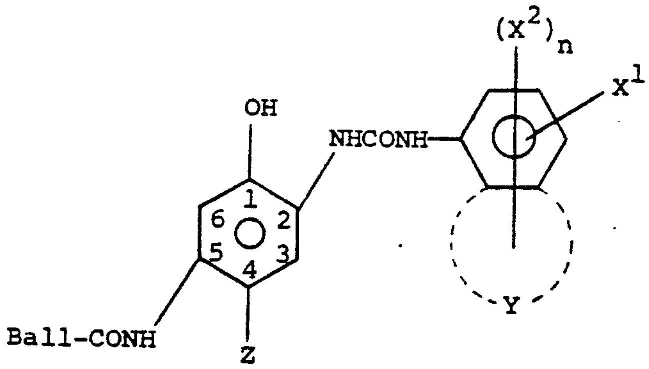

dans laquelle

$X^1$ représente $—COOR^1$, $—COR^1$, $—SO_2OR^1$, $—SO_2R^1$, 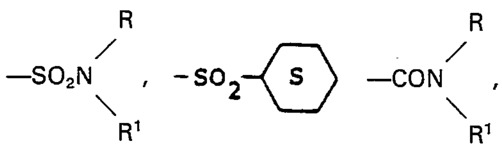

$—NO_2$ or $—CF_3$,

$R^1$ étant un groupe alkyle ou aryle éventuellement substitué et R étant un atome d'hydrogène ou un groupe alkyle ou aryle, éventuellement substitué; ou R et $R^1$ forment ensemble un cycle à 5 ou 6 membres;
$X^2$ est un atome d'halogène, un groupe hydroxyle, un groupe nitro ou un groupe alkyle, alkylcarbonyloxy, arylcarbonyloxy, alkoxy, aryloxy, alkylcarbonyle ou arylcarbonyle, éventuellement substitué;
Y, lorsqu'il est présent, est un groupe d'atomes non-métalliques qui complète un cycle condensé à 5 ou 6 membres;
Z est un atome d'hydrogène ou un atome ou groupe amovible par couplage avec un produit d'oxydation d'un agent de développement couleur;
Ball est un groupe de ballast; et
n est égal à zéro ou à un nombre entier de 1 à 4 tel que, lorsque
n est égal ou supérieur à 2, chaque $X^2$ puisse être identique ou différent.

2. Matériau photosensible à base d'halogènure d'argent selon la revendication 1, caractérisé en ce que le couplant bleu-vert du type phénolique répond à la formule

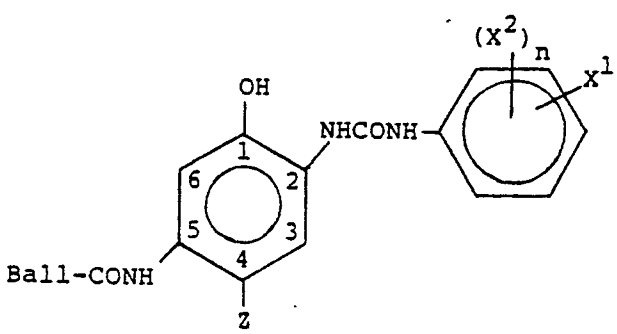

dans laquelle:
$X^1$, $X^2$, Z, Ball et n ont les mêmes significations que données à la revendication 1.

3. Matériau photosensible à base d'halogénure d'argent selon la revendication 2, caractérisé en ce que le groupe Ball—CONH est un groupe alkylcarbonylamino ou arylcarbonylamino.

4. Matériau photosensible à base d'halogénure d'argent selon l'une quelconque des revendications 1 à 3, caractérisé en ce que n est égal à zéro.

5. Matériau photosensible à base d'halogénure d'argent selon la revendicaiton 1, caractérisé en ce que le couplant bleu-vert du type phénolique répond à la formule:

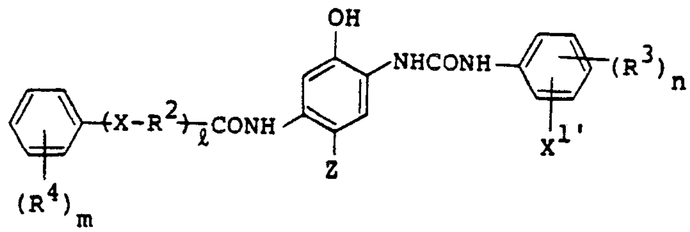

dans laquelle
X est un atome d'oxygéne ou un atome de soufre;
$R^2$ est un groupe alkylène en $C_1$—$C_{20}$ liniaire ou ramifié;
$R^3$ est un atome d'hydrogène, un atome d'halogène ou un groupe alkyl, alkylcarbonyloxy, arylcarbonyloxy, alkoxy, aryloxy, alkylcarbonyle our arylcarbonyle éventuellement substitué,
$R^4$ est un atome d'hydrogène ou d'halogène, un groupe hydroxy ou un groupe alkyle, aryle, hétérocyclique, aralkyle, alkoxy, aryloxy, alkylcarbonyloxy, arylcarbonyloxy, carboxy, alkoxycarbonyle, aryloxycarbonyle, mercapto, alkylthio, arylthio, alkylsulfonyle, arylsulfonyle, alkylcarbonyle, arylcarbonyle, alkylcarbonylamino, arylcarbonylamino, sulfonoamido, carbamoyle ou sulfamoyle éventuellement substitué;

$X^1$ représente $-COOR^1$, $-COR^1$, $-SO_2OR^1$, $-SO_2R^1$, $-SO_2N{<}^{R}_{R^1}$, $-CON{<}^{R}_{R^1}$, $-NO_2$ or $-CF_3$,

où R est un atome d'hydrogène, un groupe alkyle ou aryle et $R^1$ est un groupe alkyle ou un groupe aryle;
n est égal à 0 ou un nombre entier de 1 à 3;
m est un nombre entier de 1 à 4;
l est égal à 0 ou 1, et Z a la même signification que donnée à la revendication 1.

6. Matériau photosensible à base d'halogeńure d'argent selon les revendications 1, 3 ou 4, caractérisé en ce que $X^1$ représente

$-COOR^1$, $-COR^1$, $-SO_2OR^1$, $-SO_2R^1$, $-CON{<}^{R}_{R^1}$, $-NO_2$ or $-CF_3$,

où $R^1$ et R ayant les mêmes significations que données à la revendication 1.

7. Matériau photosensible à base d'halogénure d'argent selon l'une quelconque des revendications 1 à 3, caractérisé en ce que $X^1$ est $-SO_2R^1$, $R^1$ ayant la même signfication que donnée à la revendication 1.

8. Materiau photosensible à base d'halogénure d'argent selon la revendication 1, caractérisé en ce que $R^1$ est un groupe alkyle ou aryle, R est un atome d'hydrogène ou un groupe alkyle ou aryle et Z est un atome d'hydrogène ou un groupe amovible par couplage avec un produit d'oxydation d'un agent de développement couleur.

9. Emulsion photosensible d'halogénure d'argent contenant un couplant bleu-vert du type phénoligne, défini selon l'une quelconque des revendications 1 à 8.

10. Elément photosensible comprenant une émulsion selon la revendication 9, déposée sur une base.

11. Couplant bleu-vert du type phénolique défini selon l'une quelconque des revendications 1 à 8.